Europäisches Patentamt

European Patent Office

Office européen des brevets

(11) Numéro de publication : **0 159 950**
**B1**

## FASCICULE DE BREVET EUROPÉEN

(45) Date de publication du fascicule du brevet :
08.07.87

(51) Int. Cl.⁴ : **C 12 M 1/16**

(21) Numéro de dépôt : **85400782.0**

(22) Date de dépôt : **19.04.85**

(54) Dispositif pour effectuer des prélèvements dans des milieux semi-solides.

(30) Priorité : **19.04.84 FR 8406203**

(43) Date de publication de la demande :
**30.10.85 Bulletin 85/44**

(45) Mention de la délivrance du brevet :
**08.07.87 Bulletin 87/28**

(84) Etats contractants désignés :
**AT BE CH DE FR GB IT LI LU NL SE**

(56) Documents cités :
**EP-A- 0 086 706**
**DE-A- 3 043 419**
**GB-A- 1 361 401**
**GB-A- 2 067 126**
**US-A- 4 341 735**

(73) Titulaire : **RHONE-POULENC SANTE**
**Les Miroirs 18 Avenue d'Alsace**
**F-92400 Courbevoie Cedex (FR)**

(72) Inventeur : **Barrere, Geneviève**
**50, rue des Ecoles**
**F-75005 Paris (FR)**
Inventeur : **Beydon, Marie-Hélène**
**30 boulevard du Temple**
**F-75011 Paris (FR)**
Inventeur : **Drugeault, Lionel**
**13 rue de la République**
**F-94220 Charenton (FR)**
Inventeur : **Vasseur, Jean-Pierre**
**19 Square Maurice Ravel**
**F-91160 Longjumeau (FR)**

(74) Mandataire : **Pilard, Jacques et al**
**RHONE-POULENC RECHERCHES Service Brevets**
**Pharma 25, Quai Paul Doumer**
**F-92408 Courbevoie Cedex (FR)**

## Description

La présente invention concerne un dispositif automatique permettant de prélever une carotte dans un milieu semi-solide et de la déposer sur un autre milieu tout en lui conservant ses caractéristiques.

Le dispositif selon l'invention est particulièrement utile pour effectuer des prélèvements dans des milieux de culture gélosés employés en microbiologie et en particulier pour mettre en œuvre la méthode A.P.M. (Agar Piece Method) décrite, par exemple, par T. Ichikawa et coll., Folia Microbiol., 16, 218 (1971).

La technique A.P.M. consiste à prélever à l'aide d'un emporte-pièce calibré une carotte cylindrique dans un milieu nutritif gélosé où se développe un microorganisme. Ce cylindre de gélose est ensuite déposé sur un autre milieu semi-solide qui permet de détecter la présence d'un produit actif dans le cylindre et éventuellement de le doser. Le produit actif peut être :

soit un métabolite (a) ou un antimétabolite (b) synthétisé par le microorganisme dont la révélation peut être effectuée par voie microbiologique et/ou enzymatique et/ou chimique. Par exemple, dans le cas de la révélation microbiologique le milieu gélosé de révélation peut contenir un germe test qui exige pour sa croissance la présence d'un métabolite (a) ou qui est sensible à l'antimétabolite (b) étudié. Après incubation, le diamètre des auréoles d'exhibition ou d'inhibition de croissance du microorganisme test renseigne sur la quantité de produit actif contenu dans la carotte de gélose,

soit un composant du milieu de culture dont on veut suivre l'évolution au cours de la croissance du microorganisme, par exemple un substrat carboné, un substrat azoté, un métabolite agissant au niveau de la biosynthèse du produit étudié (précurseur, facteur de régulation, antimétabolite). Comme précédemment, la révélation peut être effectuée par voie microbiologique, enzymatique ou chimique.

La méthode A.P.M. est particulièrement utile, par exemple, pour sélectionner des souches microbiennes (amélioration de la productivité, amélioration de la qualité d'une substance active déterminée) ou pour détecter de nouvelles activités.

Jusqu'à présent, les dispositifs utilisés au laboratoire pour mettre en œuvre la méthode A.P.M. sont des emporte-pièces manuels munis d'un mandrin co-axial. L'emporte-pièce est un cylindre creux dont le diamètre intérieur est par exemple de 4 mm et le diamètre extérieur de 6 mm et dont l'extrémité est biseautée vers l'extérieur. Le prélèvement est assuré en découpant la carotte à l'aide de l'emporte-pièce puis en effectuant un mouvement d'inclinaison pour décoller la carotte du fond de la boîte, puis en relevant l'emporte-pièce. La carotte ainsi prélevée est ensuite déposée sur la surface d'un autre milieu gélosé convenable en la repoussant à l'aide du mandrin dont le diamètre est légèrement inférieur au diamètre intérieur de l'emporte-pièce de façon à pouvoir coulisser facilement. En utilisant un tel dispositif, les carottes obtenues ont des formes non reproductibles variant avec l'angle d'attaque au moment du découpage.

Il est particulièrement intéressant de pouvoir automatiser le plus possible la méthode A.P.M. pour améliorer la reproductibilité et pour effectuer rapidement un grand nombre de manipulations, ce qui est en particulier souhaitable pour sélectionner efficacement des souches.

Dans les brevets anglais GB 1 361 401 et GB 2 067 126 et européen EP 86 706 sont décrits des dispositifs pour réaliser des cavités dans une substance semi-solide, mais ces dispositifs ne permettent pas de récupérer et d'utiliser la substance semi-solide prélevée.

Il a maintenant été trouvé, et c'est ce qui fait l'objet de la présente invention, que l'on peut effectuer automatiquement le prélèvement et la dépose des carottes de milieu semi-solide au moyen d'un dispositif décrit plus complètement ci-après.

Le dispositif selon l'invention est constitué :

d'un manipulateur à 3 degrés de liberté (X, Y, Z)

d'un porte-outils

des outils emporte-pièces

d'un ensemble d'électro-vannes pour l'admission des fluides (air comprimé, vide)

éventuellement d'un système de nettoyage des outils, et

d'un organe de commande gérant l'ensemble.

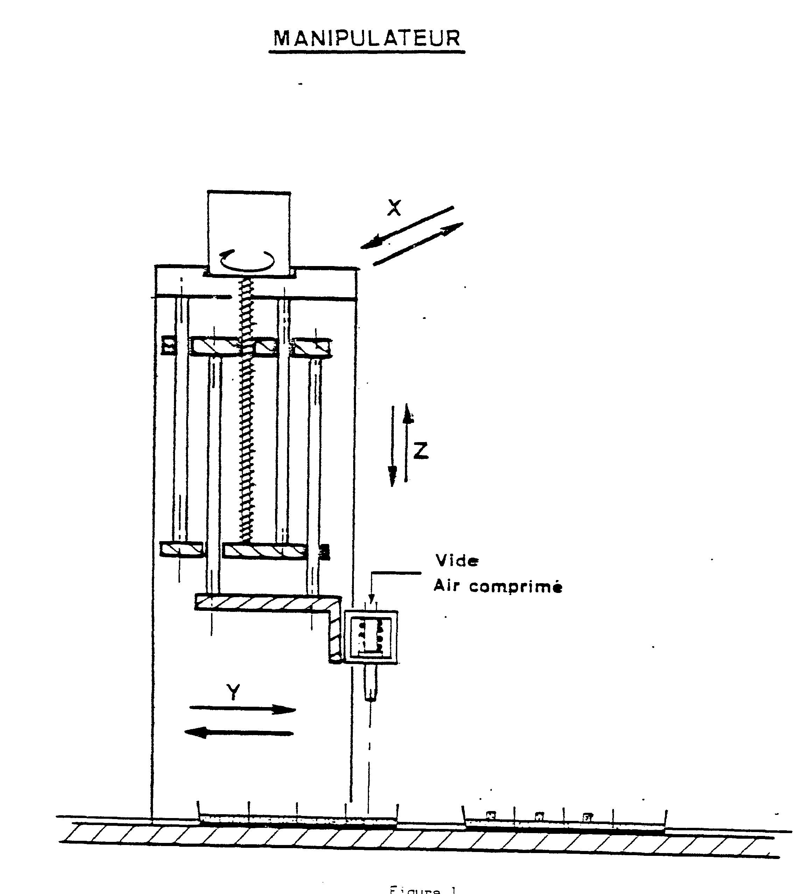

Le manipulateur, représenté schématiquement par la figure 1, peut être déplacé selon un repère orthogonal (O, X, Y, Z) grâce à 3 moteurs qui sont commandés par un microprocesseur qui en fonction d'ordres extérieurs génère les signaux électriques propres à effectuer ces déplacements. Ces ordres permettent le positionnement du manipulateur en n'importe quel point de repère (O, X, Y, Z). Ces commandes sont, soit des ordres de déplacement absolu, soit des ordres de déplacement relatif.

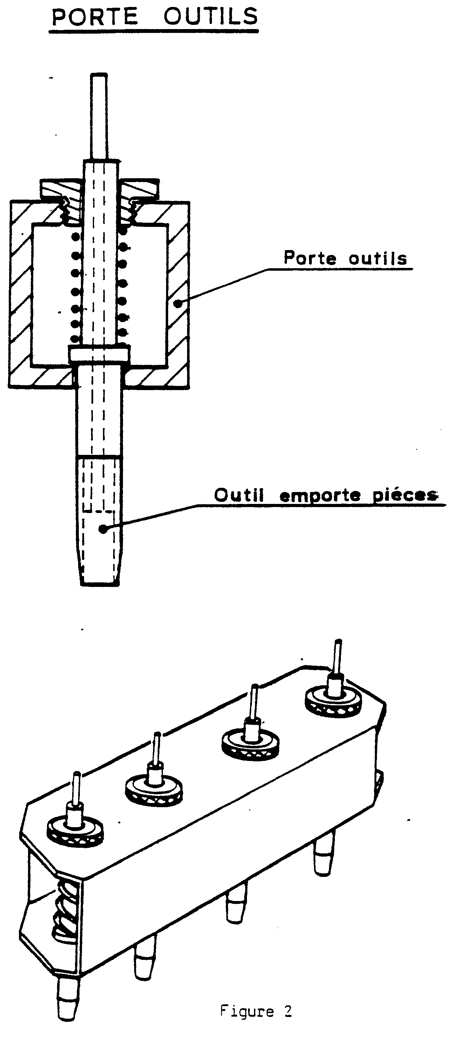

A l'extrémité du manipulateur est fixé, de façon simple et rapide, un porte-outils, représenté schématiquement par la figure 2, pouvant recevoir plusieurs emporte-pièces. Généralement, pour des raisons de commodité et de rapidité d'exécution compte tenu des dimensions des boîtes de Petri utilisées, on utilise 4 emporte-pièces. Cependant il est possible d'augmenter ou de réduire le nombre de ces outils.

Un emporte-pièce selon l'invention, est un cylindre creux, généralement en acier inoxydable, dont l'extrémité inférieure est biseautée et dont la partie supérieure est traversée par un tube creux coaxial dont le diamètre est inférieur ou égal au diamètre intérieur de l'emporte-pièce qui est relié alternativement à une source d'air comprimé et à un générateur de vide et qui délimite à la partie inférieure une cavité cylindrique dont la hauteur est supérieure à la moitié du diamètre intérieur de l'emporte-pièce. Vers la partie supérieure de l'emporte-pièce se trouve un dispositif de fixation et de positionnement sur le porte-outils qui peut être une collerette en acier inoxydable ou en téflon.

Le diamètre intérieur de l'emporte-pièce peut varier dans de larges limites. Par exemple, le diamètre intérieur de l'emporte-pièce peut être de 4 mm pour effectuer des prélèvements de carottes dont la hauteur sera voisine de 4 mm.

La hauteur utile à l'intérieur de l'emporte-pièce est supérieure à l'épaisseur du milieu semi-solide dans lequel le prélèvement est effectué.

Pour que le décollement de la carotte de milieu semi-solide soit facilité et pour éviter la détérioration du milieu semi-solide au cours du prélèvement, il est avantageux de favoriser le passage de l'air sous la carotte à prélever au moment et pendant le prélèvement.

A cet effet, il est possible :

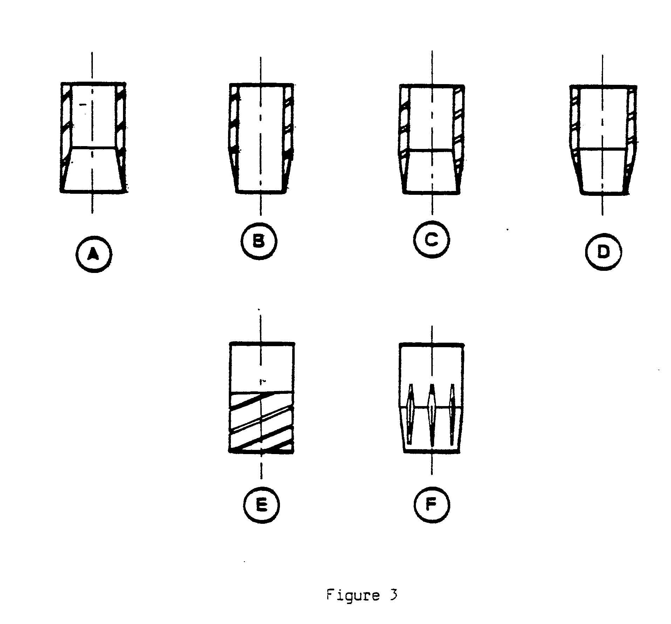

de biseauter la partie inférieure de l'emporte-pièce vers l'intérieur du cylindre (figure 3A)

de biseauter la partie inférieure de l'emporte-pièce vers l'extérieur du cylindre (figure 3B)

de biseauter la partie inférieure de l'emporte-pièce à la fois vers l'intérieur et vers l'extérieur du cylindre (figures 3C et 3D).

La partie extérieure de la zone biseautée peut être parfaitement lisse ou creusée de rainures le long des génératrices du cône (figure 3E) ou de sillons hélicoïdaux allant jusqu'à l'extrémité inférieure de l'emporte-pièce (figure 3F). Cependant les meilleurs résultats sont obtenus lorsque la partie inférieure de l'emporte-pièce est biseautée vers l'extérieur avec une paroi parfaitement lisse.

Généralement, la hauteur de la partie biseautée est égale ou supérieure à l'épaisseur du milieu semi-solide dans lequel le prélèvement est effectué.

Par ailleurs, l'angle de biseautage doit être suffisant pour que, au cours de la pénétration de l'emporte-pièce dans le milieu semi-solide, le milieu semi-solide, du fait de sa consistance, ne colle pas sur la partie biseautée créant ainsi un espace favorisant l'arrivée de l'air au fond du récipient contenant le milieu semi-solide.

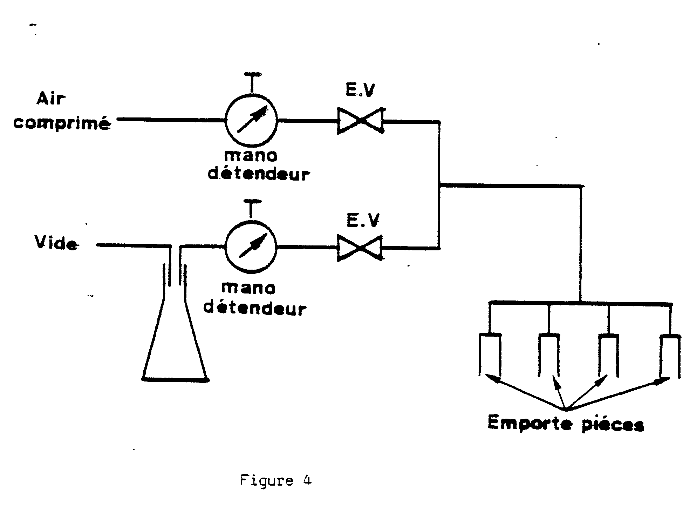

Le tube à la partie supérieure de l'emporte-pièce est relié par un tuyau souple, par exemple en silicone, à une source d'air comprimé et à un générateur de vide par l'intermédiaire de deux électro-vannes de telle manière qu'il soit possible de créer, alternativement, une dépression voisine de — 50 mm de mercure ou une surpression voisine de 100 mm de mercure (figure 4). Les électro-vannes sont commandées par le même système que celui qui génère les déplacements du manipulateur afin de rester synchrone avec ces déplacements.

L'emporte-pièce peut, au cours des opérations de prélèvement, être contaminé par des substances qui se déposent sur sa paroi aussi bien interne qu'externe. Ces substances peuvent avoir une influence sur les propriétés des carottes de gélose prélevées ultérieurement et conduire à une modification des résultats de l'étude effectuée. Pour remédier à ces inconvénients, il est avantageux de nettoyer l'outil de carottage après chaque prélèvement au moyen d'un dispositif approprié.

L'organe de commande qui permet de piloter les différents organes du dispositif (manipulateur, électro-vannes) est un ordinateur programmé pour générer les différents mouvements.

Selon l'invention, le prélèvement et la dépose d'une carotte d'un milieu semi-solide s'effectuent en réalisant la succession des étapes suivantes : (dans un but de simplification il ne sera question dans ce qui suit que des déplacements d'un emporte-pièce, les autres outils pouvant être contenus dans le dispositif se déplaçant de manière identique):

1) le manipulateur positionne l'emporte-pièce au-dessus du milieu semi-solide dans lequel le prélèvement doit être effectué.

2) l'emporte-pièce descend et pénètre de 1 mm dans le milieu semi-solide ; une dépression de — 50 mm de mercure est créée par ouverture de l'électro-vanne liée au générateur de vide.

3) l'emporte-pièce descend jusqu'à entrer en contact avec le fond du récipient contenant le milieu semi-solide, la vitesse du moteur actionnant la descente de l'outil étant réduite au minimum de telle manière que le moteur puisse patiner lorsque l'outil est en contact avec le fond du récipient.

4) l'emporte-pièce remonte d'environ 1 mm au-dessus du fond du récipient puis subit un déplacement horizontal selon un mouvement carré de 2 mm d'amplitude de façon à faire pénétrer l'air sous la carotte et à supprimer ainsi l'effet de ventouse qui empêcherait la carotte de remonter. Ce mouvement permet de réduire la dépression régnant à l'intérieur de la carotte, évitant à celle-ci d'être endommagée.

5) l'outil remonte jusqu'à la surface du milieu semi-solide et l'électro-vanne liée au générateur de vide est fermée.

6) l'outil remonte alors complètement et est prêt pour l'opération de dépose.

7) le manipulateur positionne l'emporte-pièce au-dessus du récipient contenant le milieu sur lequel la carotte doit être déposée.

8) l'outil descend jusqu'à une distance de 2 mm au-dessus du milieu semi-solide et l'électro-vanne liée au générateur d'air comprimé est alors ouverte permettant ainsi à la carotte d'entrer en contact avec le milieu semi-solide. L'électro-vanne liée au générateur d'air comprimé est alors fermée de façon à éviter de souffler de l'air à la surface du milieu semi-solide.

9) l'emporte-pièce remonte lentement, la carotte restant posée sur la gélose, puis est dégagé complètement et il est alors positionné au-dessus du poste de nettoyage. Après nettoyage, l'emporte-pièce est prêt pour une nouvelle opération de prélèvement et de dépose d'une carotte.

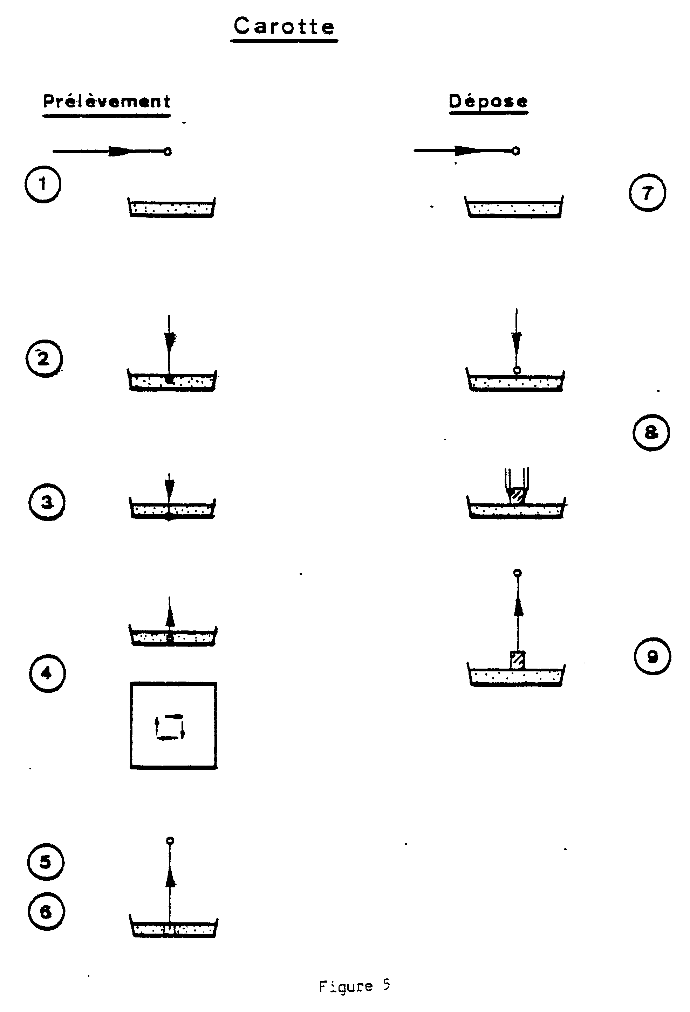

La figure 5 représente schématiquement les opérations de prélèvement et de dépose d'une carotte.

Le dispositif selon l'invention peut trouver son application, par exemple, à la sélection de souches améliorées pour la production d'un antibiotique ou d'un facteur de croissance, à la recherche de mutants surproducteurs d'activité enzymatique extracellulaire ou à la détection de nouveaux métabolites présentant une activité thérapeutique.

Les exemples suivants, donnés à titre non limitatif, illustrent l'application du dispositif selon l'invention.

Exemple 1. Sélection de souches améliorées

A l'aide du dispositif qui fait l'objet de la présente invention, on prélève des carottes dans un milieu nutritif gélosé, contenu dans une boîte de Petri, sur lequel est réalisée la culture du microorganisme Streptomyces ambofaciens producteur de spiramycine.

Les carottes sont déposées sur des boîtes de Petri contenant un milieu gélosé ensemencé dans sa masse avec des spores de Bacillus subtilis qui est un germe sensible à la spiramycine.

La spiramycine contenue dans les carottes diffuse dans le milieu. Après une incubation à 37 °C qui permet l'obtention d'une croissance confluente du germe-test Bacillus subtilis, on observe des auréoles d'inhibition de croissance par la spiramycine autour des carottes.

L'existence d'une relation linéaire entre le carré du diamètre de l'auréole et le logarithme de la concentration en spiramycine dans la carotte permet de comparer les productions de différentes souches et de repérer les souches mutantes surproductrices ou non-productrices.

Exemple 2. Recherche de mutants surproducteurs d'activité enzymatique extracellulaire

A l'aide du dispositif qui fait l'objet de la présente invention, on prélève des carottes qui sont déposées à la surface d'un milieu contenant le substrat d'une enzyme déterminée.

Un test approprié permet de révéler la dégradation éventuelle du substrat autour de la carotte. Par exemple, dans le cas de celluloses produites par différents microorganismes tels que Trichoderma reesei ou Clostridium thermocellum, la présence de l'enzyme se manifeste par l'apparition d'une zone claire autour de la carotte due à la dégradation de la cellulose qui opacifie le milieu.

Exemple 3. Détection de nouveaux métabolites à activité thérapeutique

Des dilutions appropriées de suspensions aqueuses d'échantillons naturels (sols, sédiments marins, ...) sont étalées sur des plaques de gélose nutritive stérile en boîte de Petri.

Ces boîtes sont incubées à une température convenable jusqu'à l'obtention de colonies microbiennes suffisamment développées et bien isolées.

Les colonies choisies pour l'expérimentation sont prélevées et transférées de façon ordonnée sur des plaques de gélose nutritive vierge en boîte de Petri.

Après incubation à une température convenable, des carottes sont prélevées à l'aide du dispositif qui fait l'objet de la présente invention et sont déposées sur divers milieux-tests révélateurs des activités thérapeutiques recherchées.

Par exemple, pour la recherche de nouveaux antibiotiques, on peut déposer les carottes sur des milieux ensemencés avec les germes Staphylococcus aureus 209 P, Escherichia coli ATCC 9637, Bacillus subtilis ATCC 6633 ou Candida tropicalis.

**Revendications**

1. Un dispositif pour effectuer automatiquement le prélèvement et la dépose de carottes de milieu semi-solide caractérisé en ce qu'il est constitué :

a) d'un manipulateur à 3 degrés de liberté dans un système orthogonal O, X, Y, Z,

b) d'un porte-outils fixé à l'extrémité du manipulateur,

c) d'un ou plusieurs outils emporte-pièces dont chacun d'eux est constitué d'un cylindre creux

dont la partie inférieure est biseautée

dont la partie supérieure est traversée par un tube creux coaxial dont le diamètre extérieur est inférieur ou égal au diamètre intérieur de l'emporte-pièce et dont le canal central est relié alternativement à une source d'air comprimé ou à un générateur de vide et qui délimite à la partie inférieure de l'emporte-pièce une cavité cylindrique dont la hauteur est supérieure à la moitié du diamètre intérieur de l'emporte-pièce,

d) d'un ensemble d'électro-vannes permettant la circulation des fluides,

e) éventuellement d'un système de nettoyage du ou des outils,

f) d'un ensemble de commandes gérant les mouvements des différents organes,
et qui permet la réalisation des étapes successives suivantes :

a) positionnement de l'emporte-pièce au-dessus du milieu semi-solide dans lequel le prélèvement doit être effectué,

b) descente et pénétration de l'emporte-pièce à une profondeur voisine de 1 mm dans le milieu semi-solide et création d'une dépression à l'intérieur de l'emporte-pièce par ouverture de l'électro-vanne liée au générateur de vide,

c) descente de l'emporte-pièce jusqu'à son entrée en contact avec le fond du récipient contenant le milieu semi-solide,

d) remontée de l'emporte-pièce à environ 1 mm du fond du récipient et réalisation d'un déplacement horizontal selon un mouvement carré de 2 mm environ d'amplitude de façon à faire pénétrer l'air sous la carotte,

e) remontée de l'emporte-pièce à la surface du milieu semi-solide et fermeture de l'électrovanne liée au générateur de vide,

f) remontée complète de l'emporte-pièce,

g) positionnement de l'emporte-pièce au-dessus du milieu sur lequel la carotte doit être déposée,

h) descente de l'emporte-pièce jusqu'à une distance de 2 mm environ au-dessus du milieu semi-solide et ouverture de l'électro-vanne liée au générateur d'air comprimé,

i) mise en contact de la carotte sous l'action de l'air comprimé avec la surface du milieu semi-solide et coupure de l'électro-vanne liée au générateur d'air comprimé,

j) remontée lente de l'emporte-pièce, la carotte restant posée sur le milieu semi-solide, jusqu'à dégagement complet de l'emporte-pièce.

2. Dispositif selon la revendication 1 caractérisé en ce qu'un emporte-pièce entrant dans la constitution du dispositif est biseauté à sa partie inférieure vers l'extérieur du cylindre.

3. Dispositif selon la revendication 1 caractérisé en ce qu'un emporte-pièce entrant dans la constitution du dispositif est biseauté à sa partie inférieure vers l'extérieur du cylindre et est creusé de rainures le long de la génératrice du cône ou d'un sillon hélicoïdal allant jusqu'à l'extrémité ouverte de l'emporte-pièce.

4. Dispositif selon la revendication 1 caractérisé en ce qu'un emporte-pièce entrant dans la constitution du dispositif est biseauté à sa partie inférieure vers l'intérieur du cylindre.


## Claims

1. Device for automatically extracting and depositing core samples from a semisolid medium, characterized in that it comprises :

a) a manipulator with 3 degrees of freedom in an orthogonal system O, X, Y, Z,

b) a toolholder fastened to the end of the manipulator,

c) one or more punching tools, each of which consists of a hollow cylinder, the lower part of which is bevelled and the upper part of which has passing through it a coaxial hollow tube, the outside diameter of which is less than or equal to the inside diameter of the punch and the central channel of which is connected alternately to a compressed-air source or to a vacuum generator and which, in the lower part of the punch, delimits a cylindrical cavity of a height greater than half the inside diameter of the punch,

d) a set of solenoid valves allowing fluids to circulate,

e) if appropriate, a system for cleaning the tool or tools,

f) a set of controls governing the movements of the various members
and making it possible to carry out the following successive steps :

a) positioning the punch above the semisolid medium from which the extraction is to take place,

b) lowering the punch so that it penetrates into the semisolid medium to a depth in the region of 1 mm and generating a vacuum inside the punch by opening the solenoid valve connected to the vacuum generator,

c) lowering the punch until it comes into contact with the bottom of the vessel containing the semisolid medium,

d) raising the punch to approximately 1 mm from the bottom of the vessel and executing a horizontal shift in a square movement over a distance of approximately 2 mm, to allow air to penetrate under the core sample,

e) raising the punch to the surface of the semisolid medium and closing the solenoid valve connected to the vacuum generator,

f) completely raising the punch,

g) positioning the punch above the medium on which the core sample is to be deposited,

h) lowering the punch to a distance of approximately 2 mm above the semisolid medium and opening the solenoid valve connected to the compressed-air generator,

i) bringing the core sample into contact with the surface of the semisolid medium under the action of the compressed air and closing the solenoid valve connected to the compressed-air generator, and

j) raising the punch slowly, the core sample remaining deposited on the semisolid medium, until the punch is completely free.

2. Device according to Claim 1, characterized in that the punch which is a component part of the device is bevelled in its lower part towards the outside of the cylinder.

3. Device according to Claim 1, characterized in that a punch which is a component part of the device is bevelled in its lower part towards the outside of the cylinder and is recessed with notches along the generatrix of the cone or with a helical groove extending up to the open end of the punch.

4. Device according to Claim 1, characterized in that a punch which is a component part of the device is bevelled in its lower part towards the inside of the cylinder.


## Patentansprüche

1. Vorrichtung zur automatischen Durchführung der Entnahme und des Absetzens von Bohrkernen aus halbfestem Milieu, dadurch gekennzeichnet, daß sie besteht aus :

a) einem Steuergerät mit drei Freiheitsgraden in einem orthogonalen System O, X, Y, Z,

b) einem an dem Arm des Steuergeräts befestigten Werkzeughalter,

c) einem oder mehreren Lochstanzwerkzeugen, von denen jedes aus einem Hohlzylinder besteht
dessen unterer Bereich zugeschliffen ist
dessen Oberteil von einem koaxialen Hohlrohr durchsetzt ist, dessen Außendurchmesser kleiner oder gleich dem Innendurchmesser des Lochstanzers ist und dessen zentraler Kanal abwechselnd mit einer Druckluftquelle oder einem Vakuumerzeuger verbunden ist und der im unteren Bereich des Lochstanzers einen zylindrischen Hohlraum begrenzt, dessen Höhe größer als die Hälfte des Innendurchmessers des Lochstanzers ist,

d) einer Anordnung von Elektroventilen, die die Zirkulation von Fluiden ermöglichen,

e) gegebenenfalls einem Reinigungssystem für das oder die Werkzeuge

f) einer Antriebsanordnung, die die Bewegungen der verschiedenen Organe steuert, und daß sie die Ausführung der folgenden Schritte ermöglicht :

a) Positionierung des Lochstanzers oberhalb des halbfesten Milieus, in dem die Entnahme erfolgen soll,

b) Absenken und Eindringen des Lochstanzers in das halbfeste Milieu bis zu einer Tiefe von etwa 1 mm und Erzeugung eines Unterdrucks im Inneren des Lochstanzers durch Öffnen des mit dem Vakuumerzeuger verbundenen Elektroventils,

c) Absenken des Lochstanzers, bis er mit dem Boden des Gefäßes in Kontakt kommt, das das halbfeste Milieu enthält,

d) Anheben des Lochstanzers auf etwa 1 mm vom Boden des Gefäßes und Ausführung einer horizontalen Verschiebung entlang einer quadratischen Bahn mit etwa 2 mm Amplitude, derart, daß Luft unter den Bohrkern eintritt,

e) Anheben des Lochstanzers an die Oberfläche des halbfesten Milieus und Schließen des an den Vakuumerzeuger angeschlossenen Elektroventils,

f) vollständiges Anheben des Lochstanzers,

g) Positionieren des Lochstanzers oberhalb des Milieus, auf dem der Bohrkern abgelagert werden soll,

h) Absenken des Lochstanzers auf eine Entfernung von etwa 2 mm oberhalb des halbfesten Milieus und Öffnen des mit dem Drucklufterzeuger verbundenen Elektroventils,

i) durch Drucklufteinwirkung erfolgendes Inkontaktbringen des Bohrkerns mit der Oberfläche des halbfesten Milieus und Schließen des mit dem Drucklufterzeuger verbundenen Elektroventils,

j) langsames Anheben des Lochstanzers, wobei der Bohrkern auf dem halbfesten Milieu verbleibt, bis zur vollständigen Freigabe des Lochstanzers.

2. Vorrichtung nach Anspruch 1, dadurch gekennzeichnet, daß ein Lochstanzer, der in die Vorrichtung eingesetzt wird, in seinem unteren Bereich an der Außenseite des Zylinders zugeschliffen ist.

3. Vorrichtung nach Anspruch 1, dadurch gekennzeichnet, daß ein Lochstanzer, der in die Vorrichtung eingesetzt wird, in seinem unteren Bereich an der Außenseite des Zylinders zugeschliffen und mit Nuten entlang der Erzeugenden des Konus oder mit einer schraubenförmigen Rille ausgebildet ist, die bis zum offenen Ende des Lochstanzers reicht.

4. Vorrichtung nach Anspruch 1, dadurch gekennzeichnet, daß ein Lochstanzer, der in die Vorrichtung eingesetzt wird, in seinem unteren Bereich an der Innenseite des Zylinders zugeschliffen ist.

# MANIPULATEUR

Figure 1

# PORTE OUTILS

Porte outils

Outil emporte piéces

Figure 2

Figure 3

Figure 4

# Carotte

**Prélèvement**                    **Dépose**

Figure 5